# EUROPÄISCHE PATENTSCHRIFT

(11) **EP 0 646 618 B1**
(45) Veröffentlichungstag und Bekanntmachung des Hinweises auf die Patenterteilung: **18.12.1996**
(21) Anmeldenummer: 94113298.7
(22) Anmeldetag: 25.08.1994
(51) Int. Cl.: C08J 3/03

(54) **Herstellung von Organopolysiloxan-Microemulsionen**
Preparation of organosiloxane microemulsions
Préparation de microémulsion d'organosiloxane

(30) Priorität: 27.08.1993 DE 4328917
(43) Veröffentlichungstag der Anmeldung: 05.04.1995
(73) Patentinhaber: Wacker-Chemie GmbH, D-81737 München (DE)
(72) Erfinder: Geck, Michael, Dr., D-84489 Burghausen (DE); Lautenschlager, Hans-Jürgen, Dr., D-84533 Haiming (DE); Deubzer, Bernward, Dr., D-84489 Burghausen (DE); Stinglhammer, Petra, D-83459 Simbach (DE); Habereder, Peter, Dr., D-82152 Krailling (DE); Ullrich, Kurt, D-84489 Burghausen (DE)

(56) Entgegenhaltungen:
- EP-A- 0 138 192
- EP-A- 0 404 027
- EP-A- 0 442 098

## Beschreibung

Die vorliegende Erfindung betrifft ein Verfahren zur Herstellung von Organopolysiloxan-Microemulsionen, bei dem die Komponenten in beliebiger Reihenfolge zusammengegeben und vermischt werden, Organopolysiloxan-Microemulsionen und deren Verwendung.

Die Herstellung von feinteiligen Organopolysiloxanemulsionen und -microemulsionen ist bereits bekannt.

Nach der EP-A 138 192 wird aus Organopolysiloxan, Emulgator und Wasser in einem ersten Schritt ein Ölkonzentrat gebildet, welches in einem zweiten Schritt sehr schnell und ohne zeitliche Verzögerung in Wasser dispergiert werden muß, damit eine genügend feinteilige Dispersion erhalten wird.

Zur Herstellung transparenter Microemulsionen aminofunktioneller Organopolysiloxane ist es nach EP-A 532 256 erforderlich, in einem ersten Schritt eine Mischung aus Siliconöl und Emulgator und daraus in einem zweiten Schritt durch Zugabe von wenig Wasser eine homogene konzentrierte Mischung herzustellen, die in einem dritten Schritt mit Wasser verdünnt und in einem vierten Schritt mit Säure versetzt wird.

In US-A 5,073,593 wird die Herstellung einer Microemulsion eines speziellen aminofunktionellen Polysiloxans beschrieben, wobei eine Mischung aus Öl, nichtionogenem Emulgator und Glycol hergestellt und diese in einer zweiten Stufe mit Säure und wenig Wasser versetzt wird; daraus wird eine konzentrierte Precursoremulsion gebildet, die anschließend in Wasser dispergiert wird.

Den Verfahren nach EP-A 138 192, EP-A 532 256 und US-A 5,073,593 ist gemeinsam, daß vor der Herstellung der eigentlichen Microemulsion erst ein Konzentrat aus öl, Emulgator(en), gegebenenfalls weiteren Zusatzstoffen und wenig Wasser hergestellt werden muß, somit in allen Fällen mehrere Herstellungsschritte durchgeführt werden müssen und eine bestimmte Reihenfolge des Zusammenrührens der Microemulsionsbestandteile vorgegeben ist.

Bei der Herstellung feinteiliger Siliconemulsionen auf Basis von aminoalkylsubstituierten Polysiloxanen nach US-A 5,057,572 aus Siliconöl, Emulgator(en), Wasser und Säure ist eine Konzentratherstellung vorab zwar nicht erforderlich; es ist aber unerläßlich, die Mischung der Komponenten zur Herstellung der feinteiligen Emulsionen auf mindestens 50°C zu erhitzen.

Es war Aufgabe der vorliegenden Erfindung, stabile Organopolysiloxan-Microemulsionen durch ein einfach durchführbares Verfahren bereitzustellen, bei dem insbesondere kein Konzentrat hergestellt werden muß, keine Reihenfolge der Komponentenzugabe eingehalten werden muß und kein Erwärmen erforderlich ist.

Die Erfindung betrifft ein Verfahren zur Herstellung von Organopolysiloxan-Microemulsionen, bei dem die Komponenten
A) Organopolysiloxan in Mengen von 10 bis 40 Gew%, bezogen auf das Gesamtgewicht der Microemulsionen,
B) mindestens ein Emulgator, der Diethylenglykolmonobutylether umfaßt,
C) Wasser, gegebenenfalls
D) Cotensid und gegebenenfalls
E) Säure
in beliebiger Reihenfolge zusammengegeben und vermischt werden, mit der Maßgabe, daß ein Energieeintrag in Form von starken Scherkräften und Wärme nicht erforderlich ist.

Die Erfindung betrifft auch Organopolysiloxan-Microemulsionen, die die Komponenten
A) Organopolysiloxan in Mengen von 10 bis 40 Gew.%, bezogen auf das Gesamtgewicht der Microemulsionen,
B) mindestens ein Emulgator, der Diethylenglykolmonobutylether umfaßt,
C) Wasser, gegebenenfalls
D) Cotensid und gegebenenfalls
E) Säure enthalten.

Die erfindungsgemäß hergestellten Organopolysiloxan-Microemulsionen sind transparent bis wasserklar und weisen vorzugsweise mittlere Teilchengrößen von höchstens 50 nm, insbesondere von höchstens 20 nm, auf. Die an sich bekannten Microemulsionen sind thermodynamisch stabil und bilden sich spontan. Im Gegensatz zur Ansicht der Fachwelt ist bei der Herstellung ein Energieeintrag in Form von Wärme oder starken Scherkräften nicht erforderlich. Das Vermischen erfolgt lediglich, um die Komponenten miteinander in Berührung zu bringen und eine homogene Organopolysiloxan-Microemulsion zu erhalten. Die beim Vermischen in die Organopolysiloxan-Microemulsionen eingebrachten Energiemengen sind sehr gering. Beispielsweise kann ein Liter Microemulsion mit einem Labormagnetrührer mit einer Abgabeleistung von 2 Watt hergestellt werden, während herkömmliche Verfahren zur Herstellung von 1 Liter Organopolysiloxan-Microemulsion hohe Rührleistungen erfordern, wie Rotor/Stator-Mischer mit etwa 450 Watt Abgabeleistung oder zumindest Flügelrührer von etwa 26 Watt Abgabeleistung.

Die im erfindungsgemäßen Verfahren eingesetzten einzelnen Komponenten können aus einem einzigen Bestandteil oder einem Gemisch verschiedener Bestandteile bestehen. Beispielsweise kann ein Gemisch verschiedener Emulgatoren als Komponente B eingesetzt werden.

Die Microemulsionen sind vom Öl-in-Wasser-Typ, d.h. sie weisen eine diskontinuierliche Ölphase, welche die Organopolysiloxane A enthält, und eine kontinuierliche Wasserphase auf.

Die Organopolysiloxane (A), welche im erfindungsgemäßen Verfahren zu einer Microemulsion verarbeitet werden, enthalten vorzugsweise zumindest ein aminofunktionelles Organopolysiloxan. Vorzugsweise bestehen mindestens 50 Gew.-%, insbesondere mindestens 75 Gew.-%, des Organopolysiloxans aus aminofunktionellen Organopolysiloxanen.

Vorzugsweise werden im erfindungsgemäßen Verfahren als Organopolysiloxane A Organopolysiloxane der allgemeinen Formel (I)

RₙR'ₘSiO_{(4-n-m)/2} (I)

eingesetzt, worin
- **R**: gleiche oder verschiedene, gegebenenfalls substituierte Kohlenwasserstoffreste oder Kohlenwasserstoffoxyreste mit jeweils 1 bis 18 Kohlenstoffatomen,
- **R'**: gleiche oder verschiedene, Si-C-gebundene, polare Gruppen enthaltende substituierte Kohlenwasserstoffreste oder Hydroxylgruppen,
- **n**: eine ganze Zahl im Wert von 0, 1, 2 oder 3,
- **m**: eine ganze Zahl im Wert von 0, 1, 2 oder 3
bedeuten, und die Summe n+m einen durchschnittlichen Wert von 1,8 bis 2,2 besitzt und m so gewählt ist, daß das Polyorganosiloxan mindestens einen Rest **R'** aufweist.

Obwohl in der allgemeinen Formel I nicht aufgeführt, kann ein Teil der Reste R durch direkt an Siliciumatome gebundene Wasserstoffatome ersetzt sein. Dies ist aber nicht bevorzugt.

Vorzugsweise besitzt die Summe **n** + **m** einen durchschnittlichen Wert von 1,9 bis 2,1.

Beispiele für Kohlenwasserstoffreste **R** sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Alkenylreste, wie der Vinyl-, Allyl- und der 5-Hexen-1-ylrest; Cycloalkylreste, wie Cyclopentyl-, Cyclohexyl-, Cycloheptylreste und Methylcyclohexylreste; Arylreste, wie der Phenyl-, Naphthyl- und Anthrylund Phenanthrylrest; Alkarylreste, wie o-, m-, p-Tolylreste, Xylylreste und Ethylphenylreste; Aralkylreste, wie der Benzylrest, der alpha- und der β-Phenylethylrest.

Beispiele für substituierte Reste **R** sind Cyanalkylreste, wie der β-Cyanethylrest, und halogenierte Kohlenwasserstoffreste, beispielsweise Halogenalkylreste, wie der 3,3,3-Trifluor-n-propylrest, der 2,2,2,2',2',2'-Hexafluorisopropylrest, der Heptafluorisopropylrest, und Halogenarylreste, wie der o-, m- und p-Chlorphenylrest.

Beispiele für gegebenenfalls substituierte Kohlenwasserstoffoxyreste **R** sind über ein direkt an ein Siliciumatom gebundenes Sauerstoffatom gebundene substituierte und unsubstituierte Kohlenwasserstoffreste R gemäß den vorstehend genannten Beispielen, insbesondere Alkoxyreste mit 1 bis 18 Kohlenstoffatomen und Phenoxyreste, speziell der Methoxy-, Ethoxy-, n-Propoxy-, iso-Propoxy- und Phenoxyrest. Vorzugsweise sind höchstens 5 % der Reste **R** gegebenfalls substituierte Kohlenwasserstoffoxyreste.

Beispiele für Reste **R'** sind aminofunktionelle Kohlenwasserstoffreste, beispielsweise Aminoalkylreste, wie der γ-Aminopropylrest und der β-Aminoethyl-γ-aminopropylrest; Aminoarylreste; Si-C-gebundene, cyclische, aminofunktionelle Reste; amidofunktionelle Reste, wie der γ-Acetamidopropylrest, teil- oder vollacetylierte β-Aminoethyl-γ-aminopropylreste; Hydroxylgruppen, über eine Alkylen- oder Arylengruppe an das Siliciumatom gebundene Carbonsäure- oder Sulfonsäurereste oder deren Ester; Mercaptoalkylreste; Si-C-gebundene Kohlenwasserstoffreste, welche Epoxy-, Hydroxy-, Amido- und/oder Carboxygruppen aufweisen.

Vorzugsweise ist der Rest **R'** ein aminofunktioneller Rest.

Beispiele für bevorzugte aminofunktionelle Reste **R'** sind Reste der allgemeinen Formel (II)

-R¹-[NR²(CH₂)ₐ]_{b}NHR², (II)

wobei
- **R**^{**1**}: einen zweiwertigen C₁- bis C₁₈-Kohlenwasserstoffrest bedeutet,
- **R**^{**2**}: ein Wasserstoffatom oder einen gegebenenfalls substituierten C₁- bis C₁₈-Kohlenwasserstoffrest bedeutet,
- **a**: die Werte 2, 3, 4, 5 oder 6 hat und
- **b**: die Werte 0, 1, 2, 3 oder 4 hat.

Beispiele für die zweiwertigen C₁- bis C₁₈-Kohlenwasserstoffreste **R**^{**1**} sind gesättigte gerad- oder verzweigtkettige oder cyclische Alkylenreste wie der Methylen- und Ethylenrest sowie Propylen-, Butylen-, Pentylen-, Hexylen-, 2-Methylpropylen-, Cyclohexylen- und Octadecylenreste oder ungesättigte Alkylen- oder Arylenreste wie der Hexenylenrest und Phenylenreste, wobei der n-Propylenrest und der 2-Methylpropylenrest besonders bevorzugt sind.

Beispiele für die gegebenenfalls substituierten C₁- bis C₁₈-Kohlenwasserstoffreste **R**^{**2**} sind die für **R** angegebenen Beispiele.

In der vorstehenden allgemeinen Formel (II) bedeuten vorzugsweise
- **R**^{**1**}: einen zweiwertigen C₂- bis C₆-Kohlenwasserstoffrest,
- **R**^{**2**}: ein Wasserstoffatom, einen Methyl- oder Cyclohexylrest
- **a**: die Werte 2 oder 3 und
- **b**: die Werte 0 oder 1.

Besonders bevorzugt sind lineare Polydimethylsiloxane, die gegebenenfalls als Reste **R** neben Methylresten höchstens 5 % C₁- bis C₃-Alkoxy- oder Hydroxyendgruppen aufweisen. Vorzugsweise weisen diese Polydimethylsiloxane als Reste **R'** die Reste H₂N(CH₂)₂NH(CH₂)₃-, H₂N(CH₂)₂NHCH₂CH(CH₃)CH₂-, H₂N(CH₂)₃-, 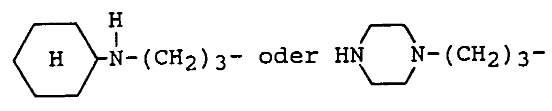 auf.

Vorzugsweise sind die Reste **R** Methyl-, Ethyl-, Phenyl-, Methoxy- und/oder Vinylreste. Wegen der leichteren Zugänglichkeit sind vorzugsweise 50 % der Reste **R**, insbesondere mindestens 80 % der Reste **R**, Methylreste.

Das im erfindungsgemäßen Verfahren eingesetzte Organopolysiloxan(gemisch) A ist vorzugsweise flüssig. Insbesondere besitzen die im erfindungsgemäßen Verfahren eingesetzten Organopolysiloxane jeweils Viskositäten von 100 mPa*s bis 50.000 mPa*s, jeweils gemessen bei 25°C.

Wird ein aminofunktionelles Organopolysiloxan A eingesetzt, so ist bevorzugt, daß es eine Aminzahl von 0,05 bis 3,0, insbesondere von 0,1 bis 1,0, besitzt. Die Aminzahl eines aminofunktionellen Stoffes wird bestimmt als Verbrauch in cm³ an 1n Salzsäure bei der Titration von 1 g des aminofunktionellen Stoffes.

Die nachstehenden Emulgatoren B eignen sich besonders für den Einsatz im erfindungungsgemäßen Verfahren:
1. Alkylsulfate, beispielsweise mit einer Kettenlänge von 8 - 18 C-Atomen, Alkylethersulfate mit 8 - 18 C-Atomen im hydrophoben Rest und 1 - 40 Ethylenoxid(EO)- bzw. Propylenoxid(PO)-Einheiten.
2. Sulfonate, z.B. Alkylsulfonate mit 8 - 18 C-Atomen, Alkylarylsulfonate mit 8 - 18 C-Atomen, Ester und Halbester der Sulfobernsteinsäure mit einwertigen Alkoholen oder Alkylphenolen mit 4 - 15 C-Atomen; gegebenenfalls können diese Alkohole oder Alkylphenole auch mit 1 - 40 EO-Einheiten ethoxyliert sein.
3. Alkali- und Ammoniumsalze von Carbonsäuren und Poly(alkylenglycol)ether-Carbonsäuren mit 8 - 20 C-Atomen im Alkyl-, Aryl-, Alkaryl- oder Aralkylrest und 1 - 40 EO- bzw. PO-Einheiten.
4. Phosphorsäureteilester und deren Alkali- und Ammoniumsalze, z.B. Alkyl- und Alkarylphosphate mit 8 - 20 C-Atomen im organischen Rest, Alkylether- bzw. Alkaryletherphosphate mit 8 - 20 C-Atomen im Alkyl- bzw. Alkarylrest und 1 - 40 EO-Einheiten.
5. Alkylpolyglycolether, vorzugsweise solche mit 2 - 40 EO-Einheiten und Alkylresten von 4 - 20 C-Atomen.
6. Alkylarylpolyglycolether mit 2 - 40 EO-Einheiten und 8 - 20 C-Atomen in den Alkyl- und Arylresten.
7. Ethylenoxid/Propylenoxid(EO/PO)-Blockcopolymere mit 8 - 40 EO- bzw. PO-Einheiten.
8. Fettsäurepolyglycolester mit 6 - 24 C-Atomen und 2 - 40 EO-Einheiten.
9. Alkylpolyglykoside der allgemeinen Formel R"-O-Zo (III), worin R" einen linearen oder verzweigten, gesättigten oder ungesättigten Alkylrest mit im Mittel 8 - 24 C-Atomen und Zo einen Oligoglykosidrest mit im Mittel o = 1 - 10 Hexose- oder Pentoseeinheiten oder Gemischen davon bedeuten.
10. Polare Gruppen enthaltende lineare Organopolysiloxane mit Alkoxygruppen und bis zu 24 C-Atomen und/oder bis zu 40 EO- und/oder PO-Gruppen.
11. Salze von primären, sekundären und tertiären Fettaminen mit 8 - 24 C-Atomen mit Essigsäure, Schwefelsäure, Salzsäure und Phosphorsäuren.
12. Quarternäre Methylalkyl- und Methylalkylbenzylammoniumsalze, deren Alkylgruppen 6 - 24 C-Atome besitzen, insbesondere die Halogenide, Sulfate, Phosphate, Acetate und Hydroxide.
13. Alkylpyridinium-, Alkylimidazolinium- und Alkyloxazoliniumsalze, deren Alkylkette bis zu 18 C-Atome besitzt, speziell in Form ihrer Halogenide, Sulfate, Phosphate und Acetate.

Der Einsatz mehrerer Emulgatoren ist bevorzugt.

Bevorzugt als Emulgatoren sind die oben unter 5., 6. und 9. aufgeführten nichtionischen Emulgatoren, ganz besonders die unter 5. aufgeführten Alkylpolyglycolether, speziell Alkylpolyglycolether mit 2 - 20 EO-Einheiten und Alkylresten von 4 - 20 C-Atomen, und die unter 9. aufgeführten Alkylpolyglykoside mit gesättigtem Alkylrest mit im Mittel 8 - 14 C-Atomen und einem mittleren Glykosidierungsgrad n zwischen 1,1 und 3.

Beispielsweise zur Verkleinerung der Teilchengröße und zur Verringerung der benötigten Menge an Emulgatoren B können im erfindungungsgemäßen Verfahren Cotenside D eingesetzt werden.

Unter Cotensiden D versteht man polare Verbindungen mittlerer Molmasse, wie Alkohole der Molekülgröße C₃ bis C₈, geeignete Di- und Polyole, Amine, Ester und Ketone.

Beispiele für besonders geeignete Cotenside D sind 1-Propanol, 2-Propanol, 1-Butanol, 2-Butanol, 2-Methyl-2-propanol, 1-Pentanol, 2-Pentanol, 3-Pentanol, 1-Hexanol, 2-Hexanol, 3-Hexanol, 1-Heptanol, 2-Heptanol, 3-Heptanol, 4-Heptanol, 1-Octanol, 2-Octanol, 3-Octanol und 4-Octanol; Glycerin, 1,2-Butandiol, 1,3-Butandiol und 1,2-Hexandiol; 1-Aminobutan, 2-Aminobutan, 2-Amino-2-methyl-propan, 1-Aminopentan, 2-Aminopentan, 1-Aminohexan, 1-Aminoheptan und 1-Aminooctan; Ethyl-, Propyl-, Isopropyl-, Butyl-, Isobutyl-, Pentyl-, Isopentyl- und Hexylacetat; Methyl-, Ethyl- und tert.-Butylpropionat; Methyl-, Ethyl-, Propyl- und Butylbutyrat; 2-Butanon, 2-Pentanon, 3-Pentanon, 4-Methyl-2-pentanon, 2-Hexanon, 3-Hexanon, 2-Heptanon, 3-Heptanon, 4-Heptanon, 5-Methyl-3-Heptanon, 2-Octanon und 3-Octanon.

Beispiele für bevorzugte Cotenside D sind 1-Alkanole der vorstehend aufgeführten Beispiele mit C₅- bis C₈-Ketten, Glycerin, Propyl-, Butyl- und Pentylacetat sowie 2-Pentanon.

Als Cotenside D besonders bevorzugt sind 1-Pentanol, 1-Hexanol, 1-Octanol und Glycerin.

Im erfindungungsgemäßen Verfahren können Säuren E zur Einstellung eines gewünschten pH-Werts oder zur Bildung von Säureadditionssalzen mit einer anderen Komponente eingesetzt werden. Bevorzugt ist der Einsatz von Säuren, wenn Organopolysiloxane A der vorstehenden allgemeinen Formel (I) verwendet werden, in der **R'** aminofunktionelle Reste bedeutet.

Beispiele für Mineralsäuren, die sich beispielsweise mit den vorstehend genannten aminofunktionellen Kohlenwasserstoffresten **R'** zu den entsprechenden ammoniumfunktionellen Resten umsetzen lassen, sind Salz-, Perchlor-, Schwefel-, schweflige, Salpeter-, salpetrige, Fluß-, Phosphor-, Diphosphor- und Polyphosphorsäuren. Beispiele für geeignete Carbonsäuren sind Ameisen-, Essig-, Propion-, Butansäuren, Citronensäure, Trichlor-, Dichlor- und Chloressigsäure, Trifluoressigsäure, Cyanessigsäure, Phenylessigsäure, Benzoesäure, m- und p-Nitrobenzoesäure, Oxalsäure, Malonsäure und Milchsäure.

Besonders bevorzugt sind Essigsäure und Ameisensäure.

Bei dem erfindungsgemäßen Verfahren können neben den Komponenten Organopolysiloxan A, Emulgator B, Wasser C, Cotensid D und Säure E noch Zusatzstoffe F eingesetzt werden. Die Zusatzstoffe F sind insbesondere Bactericide, Fungicide, Algicide, Mikrobicide, Duftstoffe, Korrosionsinhibitoren, Farbstoffe, Pigmente, Verdickungsmittel und Füllstoffe.

Die Anteile des Organopolysiloxans A als Ölphase in der kontinuierlichen Wasserphase können variiert werden, je nachdem, welcher Festgehalt in den Mikroemulsionen angestrebt wird. Vorzugsweise werden an Emulgator B 1 bis 30 Gew.-%, insbesondere 2 bis 25 Gew.-%, an Cotensid D 0 bis 15 Gew.-%, insbesondere 0 bis 10 Gew.-%, an Säure E 0 bis 3 Gew.-%, insbesondere 0 bis 1,5 Gew.-% und an Zusatzstoffen F 0 bis 3 Gew.-%, insbesondere 0 bis 1,5 Gew.-%, jeweils bezogen auf das Gesamtgewicht der Microemulsion, eingesetzt, wobei der Rest aus Wasser C besteht. Der Anteil an Wasser C beträgt vorzugsweise mindestens 31 Gew.-%.

Das Vermischen der Komponenten A bis F erfolgt in variabler Reihenfolge durch Rühren ohne Eintrag von Scherkräften, z. B. durch Blatt-, Balken-, Anker- und Gitterrührer, bei kleinen Ansätzen auch beispielsweise durch Glasstab oder Spatel oder durch Schütteln.

Der beim Vermischen auf die Mischung der Komponenten ausgeübte Druck ist vorzugsweise der Atmosphärendruck; die herrschende Temperatur ist vorzugsweise 10 bis 30°C, insbesondere die gegebenenfalls bei den Mischvorgängen durch thermodynamische Prozesse erhöhte oder erniedrigte (Raum-)Temperatur.

In einer bevorzugten Ausführungsform des erfindungsgemäßen Verfahrens werden aminofunktionelles Organopolysiloxan (A), Emulgator(en) (B), Wasser (C), gegebenenfalls Cotensid(e) (D), gegebenenfalls Säure (E) und Zusatzstoff(e) (F) durch Rühren oder Schütteln gemischt. Das Mischen kann in variabler Reihenfolge erfolgen. Werden mehrere Organopolysiloxane (A1, A2,...), Emulgatoren (B1, B2, ...), Cotenside (D1, D2,...), Säuren (E1, E2,...) und/oder Zusatzstoffe (F1, F2,...) eingesetzt, kann auch deren Reihenfolge in der Art variiert werden, daß z.B. Emulgator B1 mit Wasser, Öl A1, Cotensid D1, Öl A2, Emulgator B2 versetzt oder z.B. Öl A1 mit Cotensid D1, Emulgator B1, Wasser, Emulgator B2, Öl A2 versetzt wird oder andere Reihenfolgen gewählt werden. Auch das zugesetzte Wasser kann in einer Portion, es kann aber auch in mehreren Portionen an verschiedenen Stellen der Komponentenzugabe hinzugefügt werden.

Die Komponenten werden bevorzugt sukzessive zugegeben, können aber auch gleichzeitig dosiert werden. Bevorzugt wird während der Zugabe der Komponenten gerührt bzw. nach jeder Komponentenzugabe kurz geschüttelt, was aber nicht erforderlich ist; es kann auch nach Dosierung aller Komponenten erstmals Durchmischung erfolgen.

Werden die Komponenten aufeinanderfolgend zugegeben, ist das Einhalten bestimmter Rühr- oder Standzeiten nach Zugabe einzelner oder bestimmter Komponenten und/bzw. vor Zugabe weiterer Komponenten nicht erforderlich; es können unverzüglich alle Komponenten dosiert und gemischt werden. Es können aber auch Rühr- oder Standzeiten von einigen Minuten bis einigen Monaten zwischen den Zugaben der verschiedenen Komponenten liegen. Es ist bevorzugt, daß nach der Dosierung der letzten der in den Microemulsionen enthaltenen Komponenten Rühr- bzw. Standzeiten von 5 Minuten bis 24 Stunden eingehalten werden.

Die Microemulsionen können überall eingesetzt werden, wo auch bisher Siliconemulsionen und -microemulsionen eingesetzt worden sind. Besonders sind sie geeignet als Mittel oder als Bestandteil eines Mittels zur Imprägnierung von Fasern und Geweben, in Kosmetika, Reinigungs- und Poliermitteln, in Anstrichen oder Imprägniermitteln für Baustoffe und deren Vorprodukte, in Antischaummitteln und für klebrige Stoffe abweisende Beschichtungen. So können sie verwendet werden zum Schlichten von Glas-, Keramik- und Kohlefasern, zum Imprägnieren und Beschichten von Textilfasern - beispielsweise als Fadengleitmittel - und Textilgeweben, in Kosmetika, wie Handcremes, Body-Lotions, Shampoos, Haarspülungen, Haarfestigern, Rasiercremes und -lotionen, in Polituren, wie Möbel-, Fußboden- und Autopolituren, in Wachsen, wie Fußbodenwachsen und in Desinfektionsmitteln, zur Hydrophobierung von Gips vor oder nach dessen Formgebung zu Bauteilen, zum Imprägnieren von Natur- oder Kunststein, Beton, Zement, Mauerwerk, zum Hydrophobieren von Gasbeton vor oder nach dessen Aufschäumen, in Anstrichen für Bauwerke und deren Teile, wie Dispersionsfarben, insbesondere in Siliconfarben, in oder als Papierbeschichtungen für Träger von selbstklebenden Etiketten und als Formtrennmittel für Polymere.

Besonders bevorzugt ist die Verwendung der Microemulsionen, insbesondere der Microemulsionen aminofunktioneller Organopolysiloxane A, als Mittel oder in Mitteln zur Imprägnierung und Beschichtung von Textilfasern und -geweben. So verleihen die Microemulsionen den damit behandelten textilen Fasern und Flächengebilden beispielsweise einen sehr angenehmen, weichen Griff.

### Beispiele

In den nachfolgenden Beispielen sind, soweit nichts anderes angegeben ist, alle Mengen- und Prozentangaben auf das Gewicht bezogen. Falls jeweils nicht anders angegeben, werden die nachfolgenden Beispiele bei Atmosphärendruck (etwa 0,1 MPa (abs.)) und Raumtemperatur (etwa 20°C) bzw. bei Temperaturen und Drücken, die sich beim Zusammengeben der Reaktanden bei Raumtemperatur ohne zusätzliche Heizung oder Kühlung oder durch die Einwirkung von Mischorganen auf die Komponenten bzw. Mischungen einstellen, durchgeführt.

Aminofunktionelle Organopolysiloxane A:
A1:
   Organopolysiloxan bestehend aus Dimethylsiloxy-Methyl(N-[2-Aminoethyl]-3-aminopropyl)siloxy-Einheiten und endständigen Methoxydimethylsilylgruppen; Viskosität: 1200 mPa.s bei 25°C; Aminzahl: 0.6.
A2:
   Organopolysiloxan bestehend aus Dimethylsiloxy-Methyl(N-[2-Aminoethyl]-3-aminopropyl)siloxy-Einheiten und endständigen Methoxydimethylsilylgruppen; Viskosität: 1000 mPa.s bei 25°C; Aminzahl: 0.3.
A3:
   Organopolysiloxan bestehend aus Dimethylsiloxy-Methyl(N-[2-Aminoethyl]-3-aminopropyl)siloxy-Einheiten und endständigen Methoxydimethylsilylgruppen; Viskosität: 6500 mPa.s bei 25°C; Aminzahl: 0.13.
A4:
   Organopolysiloxan bestehend aus Dimethylsiloxy-Methyl(N-Cyclohexyl-3-aminopropyl)siloxy-Einheiten und endständigen Trimethylsilylgruppen; Viskosität: 1000 mPa.s bei 25°C; Aminzahl 0.3.

### Emulgatoren (B):

B1:
   Isotridecylethoxylat mit durchschnittlich 8 Ethylenoxideinheiten.
B2:
   Isotridecylethoxylat mit durchschnittlich 6 Ethylenoxideinheiten.
B3:
   n-Butylethoxylat mit 2 Ethylenoxideinheiten.
B4:
   C₈/C₁₁-Alkylpolyglykosid mit einem Glycosidierungsgrad von 1,35 (50 %ig in Wasser).

### Beispiel 1

Herstellung einer Microemulsion aus
30 Gewichtsteilen Polysiloxan A1,
10 Gewichtsteilen Emulgator B1,
15 Gewichtsteilen Emulgator B3,
45 Gewichtsteilen Wasser (C) und
0,57 Gewichtsteilen Eisessig (E).

Nach Vorlage der 1. Komponente wurden die anderen Komponenten unverzüglich aufeinanderfolgend zugegeben. Nach jeder Komponentenzugabe wurde kurz (ca. 15 Sekunden) geschüttelt bzw. gerührt. Nach Zugabe der letzten Komponente wurde noch 30 Minuten stehengelassen.

Es wurden folgende Reihenfolgen des Zusammengebens der Microemulsionsbestandteile ausgeführt:
a) Vorlage B1; Zugabe B3, C, E, A1.
b) Vorlage B1; Zugabe B3, E, A1, C.
c) Vorlage E; Zugabe A1, B1, B3, C.
d) Vorlage B1; Zugabe B3, C, A1, E.
e) Vorlage B3; Zugabe B1, A1, E, C.
f) Vorlage Al; Zugabe E, B1, B3, C.
g) Vorlage C; Zugabe B1, B3, A1, E.
h) Vorlage C; Zugabe B1, B3, E, A1.

In allen Fällen a) - h) wurden wasserklare Microemulsionen erhalten. "Teilchengrößen" konnten in keinem Fall bestimmt werden (gemessen mit dem Autosizer 2 C der Fa. Malvern), da die Teilchen zu klein (<< 10 nm) waren. Die Microemulsionen waren länger als 3 Monate lagerstabil, begannen aber nach Standzeiten von ca. 1 Woche sich leicht gelblich zu verfärben.

### Beispiel 2

Herstellung einer Microemulsion aus
15 Gewichtsteilen Polysiloxan A1,
5 Gewichtsteilen Emulgator B3,
30 Gewichtsteilen Emulgator B4 (50 %ig in Wasser),
50 Gewichtsteilen Wasser (C) und
0,28 Gewichtsteilen Eisessig (E).

Nach Vorlage der 1. Komponente wurden die anderen Komponenten unverzüglich aufeinanderfolgend zugegeben. Nach jeder Komponentenzugabe wurde kurz (ca. 15 Sekunden) geschüttelt bzw. gerührt. Nach Zugabe der letzten Komponente wurde noch 1 Stunde stehengelassen.

Es wurden folgende Reihenfolgen des Zusammengebens der Microemulsionsbestandteile ausgeführt:
a) Vorlage B4; Zugabe B3, C, E, A1.
b) Vorlage B3; Zugabe B4, E, A1, C.
c) Vorlage C; Zugabe E, B4, B3, A1.

In allen Fällen a) - c) wurden wasserklare, durch die Eigenfarbe des Emulgators B4 braungelb gefärbte Microemulsionen erhalten. Die "Teilchengrößen" betrugen in jedem Fall < 20 nm (gemessen mit dem Autosizer 2 C der Fa. Malvern). Die Microemulsionen waren länger als 3 Monate lagerstabil.

### Beispiel 3

Herstellung einer Microemulsion aus
15 Gewichtsteilen Polysiloxan A3,
15 Gewichtsteilen Emulgator B2,
5 Gewichtsteilen Emulgator B3,
65 Gewichtsteilen Wasser (C) und
0,07 Gewichtsteilen Eisessig (E).

Nach Vorlage der 1. Komponente wurden die anderen Komponenten unverzüglich aufeinanderfolgend zugegeben. Nach jeder Komponentenzugabe wurde kurz (ca. 15 Sekunden) geschüttelt bzw. gerührt. Nach Zugabe der letzten Komponente wurde noch 1 Stunde stehengelassen.

Es wurden folgende Reihenfolgen des Zusammengebens der Microemulsionsbestandteile ausgeführt:
a) Vorlage B2; Zugabe B3, C, E, A3.
b) Vorlage B2; Zugabe B3, E, A3, C.
c) Vorlage C; Zugabe E, B2, B3, A3.

In den Fällen a) und c) wurden wasserklare, im Fall b) wurde eine transparente Microemulsion erhalten. Die Microemulsionen waren länger als 3 Monate lagerstabil.

### Beispiel 4

Herstellung einer Microemulsion aus
20 Gewichtsteilen Polysiloxan A4,
15 Gewichtsteilen Emulgator B2,
10 Gewichtsteilen Emulgator B3,
55 Gewichtsteilen Wasser (C) und
0,32 Gewichtsteilen Ameisensäure (85 %ig) (E).

Nach Vorlage der 1. Komponente wurden die anderen Komponenten unverzüglich aufeinanderfolgend zugegeben. Nach jeder Komponentenzugabe wurde kurz (ca. 15 Sekunden) geschüttelt bzw. gerührt. Nach Zugabe der letzten Komponente wurde noch 30 Minuten stehengelassen.

Es wurden folgende Reihenfolgen des Zusammengebens der Microemulsionsbestandteile ausgeführt:
a) Vorlage B2; Zugabe B3, C, E, A4.
b) Vorlage B2; Zugabe B3, E, A4, C.
c) Vorlage A4; Zugabe E, B2, B3, C.

In allen Fällen a) - c) wurden klare bis leicht transparente Microemulsionen erhalten. Die "Teilchengröße" betrug in jedem Fall < 50 nm (gemessen mit dem Autosizer 2 C der Fa. Malvern).

## Patentansprüche

1. Verfahren zur Herstellung von Organopolysiloxan-Microemulsionen, bei dem die Komponenten
A) Organopolysiloxan in Mengen von 10 bis 40 Gew%, bezogen auf das Gesamtgewicht der Microemulsionen,
B) mindestens ein Emulgator, der Diethylenglykolmonobutylether umfaßt,
C) Wasser, gegebenenfalls
D) Cotensid und gegebenenfalls
E) Säure
in beliebiger Reihenfolge zusammengegeben und vermischt werden, mit der Maßgabe, daß ein Energieeintrag in Form von starken Scherkräften und Wärme nicht erforderlich ist.

2. Verfahren nach Anspruch 1, wobei als Organopolysiloxane A Organopolysiloxane der allgemeinen Formel (I)
RₙR'ₘSiO_{(4-n-m)/2} (I)
eingesetzt werden, worin
**R** gleiche oder verschiedene, gegebenenfalls substituierte Kohlenwasserstoffreste oder Kohlenwasserstoffoxyreste mit jeweils 1 bis 18 Kohlenstoffatomen,
**R'** gleiche oder verschiedene, Si-C-gebundene, polare Gruppen enthaltende substituierte Kohlenwasserstoffreste oder Hydroxylgruppen,
**n** eine ganze Zahl im Wert von 0, 1, 2 oder 3,
**m** eine ganze Zahl im Wert von 0, 1, 2 oder 3 bedeuten, und die Summe n+m einen durchschnittlichen Wert von 1,8 bis 2,2 besitzt und m so gewählt ist, daß das Polyorganosiloxan mindestens einen Rest **R'** aufweist.

3. Verfahren nach Anspruch 2, wobei
**R'** Reste der allgemeinen Formel (II)
-R¹-[NR²(CH₂)ₐ]_{b}NHR², (II)
bedeutet, in der
**R**^{**1**} einen zweiwertigen C₁- bis C₁₈-Kohlenwasserstoffrest bedeutet,
**R**^{**2**} ein Wasserstoffatom oder einen gegebenenfalls substituierten C₁- bis C₁₈-Kohlenwasserstoffrest bedeutet,
**a** die Werte 2, 3, 4, 5 oder 6 hat und
**b** die Werte 0, 1, 2, 3 oder 4 hat.

4. Organopolysiloxan-Microemulsionen, die die Komponenten
A) Organopolysiloxan in Mengen von 10 bis 40 Gew.%, bezogen auf das Gesamtgewicht der Microemulsionen,
B) mindestens ein Emulgator, der Diethylenglykolmonobutylether umfaßt,
C) Wasser, gegebenenfalls
D) Cotensid und gegebenenfalls
E) Säure enthalten.

5. Verwendung der Organopolysiloxan-Microemulsionen nach Anspruch 4 oder hergestellt nach einem der Ansprüche 1 bis 3 als Mittel oder in Mitteln zur Imprägnierung und Beschichtung von Textilfasern und -geweben.

## Claims

1. Process for the preparation of organopolysiloxane microemulsions, in which the components
A) organopolysiloxane in amounts of from 10 to 40% by weight, based on the total weight of the microemulsions,
B) at least one emulsifier which contains diethylene glycol monobutyl ether,
C) water, if appropriate
D) cosurfactant and if appropriate
E) acid.
are brought together and mixed in any desired sequence, with the proviso that the introduction of energy in the form of strong shear forces and heat is not necessary.

2. Process according to Claim 1, where the organopolysiloxanes A employed are organopolysiloxanes of the general formula (I)
RₙR'ₘSiO_{(4-n-m)/2} (I)
wherein
the Rs are identical or different, optionally substituted hydrocarbon radicals or hydrocarbonoxy radicals having in each case 1 to 18 carbon atoms,
the R's are identical or different Si-C-bonded substituted hydrocarbon radicals containing polar groups, or hydroxyl groups,
n is an integer having a value of 0, 1, 2 or 3 and
m is an integer having a value of 0, 1, 2 or 3,
and the sum of n+m has an average value of 1.8 to 2.2 and m is chosen such that the polyorganosiloxane contains at least one radical R'.

3. Process according to Claim 2, where the R's are radicals of the general formula (II)
-R¹-[NR²(CH₂)ₐ]_{b}NHR², (II)
in which
R¹ is a divalent C₁- to C₁₈-hydroiarbin radical,
R² is a hydrogen atom or an optionally substituted C₁-to C₁₈-hydrocarbon radical,
a has the values 2, 3, 4, 5 or 6 and
b has the values 0, 1, 2, 3 or 4.

4. Organopolysiloxane microemulsions which comprise the components
A) organopolysiloxane in amounts of from 10 to 40% by weight, based on the total weight of the microemulsions,
B) at least one emulsifier which contains diethylene glycol monobutyl ether,
C) water, if appropriate
D) cosurfactant and if appropriate
E) acid.

5. Use of the organopolysiloxane microemulsions according to Claim 4 or prepared according to one of Claims 1 to 3 as agents or in compositions for impregnating and coating textile fibres and textile fabrics.

## Revendications

1. Procédé de préparation de microémulsions d'organopolysiloxane, dans lequel les composants
(A) organopolysiloxane en des quantités de 10 à 40% en poids, sur base du poids total des microémulsions;
(B) au moins un émulsionnant qui comprend le diéthylèneglycolmonobutyléther;
(C) eau, facultativement;
(D) cotensioactif, et facultativement
(E) acide
sont mis en commun en ordre quelconque et mélangés, avec la condition qu'un apport d'énergie sous forme de forces de cisaillement élevées et de chaleur n'est pas nécessaire.

2. Procédé suivant la revendication 1, où on utilise comme organopolysiloxane (A), des organopolysiloxanes de formule générale (I)
RₙR'ₘSiO_{(4-n-m)/2} (I)
où
R représente un radical hydrocarbure ou un radical oxyhydrocarbure, identique ou différent, facultativement substitué, avec chaque fois 1 à 18 atomes de carbone;
R' représente un radical hydrocarbure identique ou différent, à liaison Si-C, substitué et contenant un groupe polaire, ou un groupe hydroxyle;
n représente un nombre entier de valeur 0, 1, 2 ou 3;
m représente un nombré entier de valeur 0, 1, 2 ou 3;
et la somme n+m possède une valeur moyenne de 1,8 à 2,2 et m est choisi de sorte que le polyorganosiloxane présente au moins un radical R'.

3. Procédé suivant la revendication 2,
où
R' représente un radical de formule générale (II)
-R¹-[NR²(CH₂)ₐ]_{b}NHR² (II)
où
R¹ représente un radical hydrocarbure bivalent en C₁ à C₁₈;
R² représente un atome d'hydrogène ou un radical hydrocarbure en C₁ à C₁₈ facultativement substitué;
a a la valeur 2, 3, 4, 5 ou 6, et
b a la valeur 0, 1, 2, 3 ou 4.

4. Microémulsions d'organopolysiloxane, qui contiennent les composants
(A) organopolysiloxane en des quantités de 10 à 40% en poids, sur base du poids total des microémulsions;
(B) au moins un émulsionnant qui comprend le diéthylèneglycolmonobutyléther;
(C) eau, facultativement
(D) cotensioactif, et facultativement
(E) acide.

5. Utilisation des microémulsions d'organopolysiloxane suivant la revendication 4 ou préparées suivant l'une quelconque des revendications 1 à 3, comme agent ou agents d'imprégnation et de revêtement de fibres et toiles textiles.
